# EUROPEAN PATENT SPECIFICATION

(11) **EP 2 241 322 B1**
(45) Date of publication and mention of the grant of the patent: **28.11.2012**
(21) Application number: 09707454.6
(22) Date of filing: 04.02.2009
(51) Int. Cl.: A61K 33/40, A61K 33/10, A61P 1/02, A61P 31/04, A61K 33/20

(54) **DENTAL STERILIZING WATER, METHOD FOR PRODUCING THE WATER, AND DEVICE FOR PRODUCING THE WATER**
ZAHNÄRZTLICHES STERILISIERUNGSWASSER, VERFAHREN ZUR HERSTELLUNG DIESES WASSERS UND VORRICHTUNG ZUR HERSTELLUNG DIESES WASSERS
EAU DE STÉRILISATION DENTAIRE, PROCÉDÉ DE PRODUCTION DE L'EAU, ET DISPOSITIF DE PRODUCTION DE L'EAU

(30) Priority: 08.02.2008 JP 2008029804
(43) Date of publication of application: 20.10.2010
(73) Proprietor: Perfect Perio Co., Ltd., Oyama-shi Tochigi (JP)
(72) Inventor: NOGUCHI, Munenori, Oyama-shi Tochigi 323-0804 (JP)
(74) Representative: Prüfer & Partner GbR European Patent Attorneys
(86) International application number: PCT/JP2009/000426
(87) International publication number: WO 2009/098870

(56) References cited:
- WO-A1-2007/072697
- WO-A1-2007/072697
- JP-A- 10 024 294

## Description

### TECHNICAL FIELD

The present invention relates to dental sterilizing water intended to be used for sterilizing dental caries pathogen, periodontal pathogen, or other oral bacteria, and relates to a method for producing the dental sterilizing water and an apparatus for producing the dental sterilizing water.

### BACKGROUND ART

As bacterial infectious diseases caused by bacteria found in the oral cavity, well known are dental caries, which is mainly caused by mutans streptococci, and gingivitis and periodontal disease, which are caused by periodontal pathogen.

The dental caries is a disease in which mutans streptococci ferment saccharose (sucrose) to organic acids such as lactic acid, and the organic acids dissolve tooth enamel to promote decalcification of teeth. At present, the mutans streptococci are classified into seven species. In the human oral cavity, two bacterial species, Streptococcus mutans (S. mutans) and Streptococcus sobrinus (S. sobrinus), are mainly found.

Meanwhile, the gingivitis is a disease caused by plaque adhering to gingival crevices between teeth and gingiva, and the periodontal disease is a disease in which the gingivitis is promoted to form deep crevices, called periodontal pockets, between teeth and gingiva. Recently, it has been being revealed that since periodontal pathogen are transferred everywhere in the body via blood, they not only cause the onset of various diseases, such as bacteremia, myocardial infarction, angina pectoris, bacterial endocarditis, arteriosclerosis, hypertension, pneumonia, and septicemia, but also exacerbate the symptoms of diabetes or cause premature birth or the birth of immature infants (low birthweight infants).

As pathogenic bacteria of periodontal diseases, anaerobic gram-negative bacilli and spirochete are typically known. Examples of the pathogenic bacteria include Porphyromonas gingivalis (hereinafter referred to as P. gingivalis), which is a gram-negative short bacillus, Actinobacillus actinomycetemcomitans (hereinafter referred to as A. actinomycetemcomitans), which is another gram-negative short bacillus, Prevotella intermedia, Tannerela forsythensis, Eikenella corrodens, which is a gram-negative short bacillus, Campylobacter rectus, which is a gram-negative bacterium, and Treponema denticola (hereinafter referred to as T. denticola), which is a spirochaete (spiral bacterium).

There are many methods for treating dental caries and periodontal disease, but currently, fundamental treatment methods have not yet been found. For example, ozone treatment is known as a method for treating periodontal disease, but since ozone itself is unstable, it is unclear whether the treatment can annihilate periodontal pathogen that are anaerobic pathogenic bacteria inhabiting in the deep zones of periodontal pockets. In addition, treatment with antibiotics has a risk of emergence of resistant strains and therefore is not yet sufficient as fundamental treatment of periodontal disease.

Furthermore, a dental caries treatment method, called 3DS, is known, but it needs to produce a tray that fits to individual patient's dentition, takes about 5 minutes for each bacterial eradication step owing to the agent used for the bacterial eradication, and requires repeating the bacterial eradication step several times. Therefore, the method forces to spend a long period of time on both the treatment side and the patient side and is complicated in the procedure and is also high in cost.

The bactericidal activity of sodium hypochlorite (NaClO: soda hypochlorite) is widely known conventionally, and it is also well known that the bactericidal component is hypochlorous acid (HClO) produced by hydrolysis.

It is well known that the so-called effective chlorine (free residual chlorine) largely varies in its form depending on pH. It is believed that in a pH higher than 7, hypochlorous acid having a strong bactericidal activity is changed to a hypochlorous ion (ClO⁻) form having a weak bactericidal activity, resulting in a drastic decrease in the presence ratio of hypochlorous acid. Accordingly, the pH of sterilizing water is set to 3 to 7 in which the presence ratio of hypochlorous acid is high, in conjunction with preventing, in the strongly acidic side, chlorine gas from being produced (Patent Documents 1 to 9).

At the same time, since such conventional sterilizing water is also used for, for example, sterilization of fruits and vegetables, disinfection of food manufacturing lines, cleaning of bathrooms or the like, disinfection and bleach of pool water, and sterilization of water discharged after sewage treatment, most of the effective chlorine concentrations are about several tens parts per million at the highest.

However, in such an effective chlorine concentration, even if the presence ratio of hypochlorous acid is high, the sterilizing water cannot annihilate bacteria, that is, the sterilizing water cannot permeate through cell walls present on the surfaces of bacteria to denature protein contained in the bacterial cells.

Under such circumstances, the present applicant has developed periodontal disease-treating sterilizing water including hypochlorous acid as the bactericidal component at a high concentration that can annihilate bacteria and has confirmed in a clinical test that the sterilizing water can annihilate periodontal pathogen (Patent Document 10).

The above-mentioned application (WO 2007-72697) is characterized in that tap water is forced to pass through reverse osmosis membrane, and the passed water is added with sodium chloride alone, followed by electrolysis. According to the invention, since carbon dioxide present in the air dissolves in the solvent to make the solvent weakly acidic, an effect of not requiring addition of an acid, such as hydrochloric acid or acetic acid, is achieved, and therefore sterilizing water without taste and odor can be produced even if the effective chlorine concentration is about several hundreds parts per million.

[Patent Document 1] Japanese Patent Application Laid-Open No. Hei 3-258392
[Patent Document 2] Japanese Patent Application Laid-Open No. Hei 4-131184
[Patent Document 3] Japanese Patent Application Laid-Open No. Hei 4-94788
[Patent Document 4] Japanese Patent Application Laid-Open No. Hei 6-312189
[Patent Document 5] Japanese Patent Application Laid-Open No. Hei 8-323365
[Patent Document 6] Japanese Patent Application Laid-Open No. Hei 9-262587
[Patent Document 7] Japanese Patent Application Laid-Open No. Hei 10-76270
[Patent Document 8] Japanese Patent Application Laid-Open No. Hei 10-24294
[Patent Document 9] Japanese Patent Application Laid-Open No. 2005-342702
[Patent Document 10] International Patent Application Publication No. WO 2007-72697

### DISCLOSURE OF INVENTION

### PROBLEMS TO BE SOLVED BY THE INVENTION

However, even in the above-mentioned application (WO 2007-72697), there is a problem that biofilms have to be physically destroyed by another method.

That is, most of pathogenic bacteria are protected by biofilms composed of exopolysaccharides that are produced by the bacteria themselves, rather than inhabiting as floating bacteria, and gradual proliferation continues while blocking the biological defense mechanism of the human body or antibiotics with the biofilms.

Consequently, the pathogenic bacteria in the biofilms cannot be annihilated by hypochlorous acid at a high concentration alone. This is a so-called biofilm infectious disease and is also a cause that makes the antibiotic administration therapy inefficient.

### MEANS FOR SOLVING THE PROBLEMS

The present invention has been made considering the above-described circumstances, and an object thereof is to provide dental sterilizing water that can prevent decalcification and has not only a bactericidal activity but also an activity of destroying biofilms and to provide a method and an apparatus for producing the dental sterilizing water.

A dental sterilizing water of claim 1 according to the present invention is sterilizing water for treating or preventing a dental disease by sterilizing periodontal pathogen, dental caries pathogen, or other oral bacteria, the sterilizing water having an effective chlorine concentration of 50 to 700 ppm and a pH of 6.3 to 8 and containing hypochlorous acid and sodium hydrogen carbonate, wherein the dental sterilizing water is produced as defined in the claims.

The dental sterilizing water according to the present invention may have an effective chlorine concentration of 201 to 700 ppm instead of 50 to 700 ppm and is used for treating a dental disease.

The dental sterilizing water according to the present invention may have an effective chlorine concentration of 400 to 700 ppm instead of 201 to 700 ppm.

The dental sterilizing water according to the present invention may have an effective chlorine concentration of 500 to 700 ppm instead of 201 to 700 ppm.

The dental sterilizing water according to the present invention may have an effective chlorine concentration of 50 to 300 ppm instead of 50 to 700 ppm and is used for preventing a dental disease.

The dental sterilizing water according to the present invention may have a pH of 7 to 8 instead of 6.3 to 8.

A method for producing dental sterilizing water of claim 5 according to the present invention is a method for treating or preventing a dental disease by sterilizing periodontal pathogen, dental caries pathogen, or other oral bacteria, the method having the steps of preparing an aqueous solution as a stock solution containing sodium chloride and carbon dioxide; and electrolyzing the stock solution such that the effective chlorine concentration is from 50 to 700 ppm, the pH is from 6.3 to 8, and hypochlorous acid and sodium hydrogen carbonate are produced, wherein the stock solution is prepared as defined in the claims.

In the method for producing dental sterilizing water according to the present invention, the stock solution is prepared by forcing water to pass through reverse osmosis membrane; adding sodium chloride to the passed water; and blowing carbon dioxide gas into the water or adding dry ice to the water before or after the step of adding sodium chloride or in the step of adding sodium chloride.

In another embodiment of the method for producing dental sterilizing water according to the present invention, the stock solution is prepared by adding sodium chloride to pure water or distilled water; and blowing carbon dioxide gas into the water or adding dry ice to the water before or after the step of adding sodium chloride or in the step of adding sodium chloride.

In another embodiment of the method for producing dental sterilizing water according to the present invention, the stock solution is prepared by forcing water to pass through reverse osmosis membrane; adding sodium chloride to the passed water; and increasing the partial pressure of carbon dioxide being in contact with the passed water to a level higher than that of carbon dioxide in the air before or after the step of adding sodium chloride or in the step of adding sodium chloride.

In another embodiment of the method for producing dental sterilizing water according to the present invention, the stock solution is prepared by adding sodium chloride to pure water or distilled water; and increasing the partial pressure of carbon dioxide being in contact with the pure water or the distilled water to a level higher than that of carbon dioxide in the air before or after the step of adding sodium chloride or in the step of adding sodium chloride.

In the method for producing dental sterilizing water according to the present invention, the effective chlorine concentration is from 201 to 700 ppm instead of from 50 to 700 ppm and the dental sterilizing water is used for treating a dental disease.

In the method for producing dental sterilizing water according to the present invention, the effective chlorine concentration is from 400 to 700 ppm instead of from 201 to 700 ppm.

In the method for producing dental sterilizing water according to the present invention, the effective chlorine concentration is from 500 to 700 ppm instead of from 201 to 700 ppm.

In the method for producing dental sterilizing water according to the present invention, the effective chlorine concentration is from 50 to 300 ppm instead of from 50 to 700 ppm; and the dental sterilizing water is used for preventing a dental disease.

In the method for producing dental sterilizing water according to the present invention, the pH is from 7 to 8 instead of from 6.3 to 8.

A stock solution of dental sterilizing water of claim 9 according to the present invention is a stock solution of dental sterilizing water for treating or preventing a dental disease by sterilizing periodontal pathogen, dental caries pathogen, or other oral bacteria, the stock solution containing sodium chloride and carbon dioxide; and giving an effective chlorine concentration of 50 to 700 ppm, a pH of 6.3 to 8, and production of hypochlorous acid and sodium hydrogen carbonate by electrolysis, wherein the stock solution is prepared as defined in the claims.

In the stock solution of dental sterilizing water according to the present invention, the effective chlorine concentration is from 201 to 700 ppm instead of from 50 to 700 ppm and the stock solution is used for treating a dental disease.

In the stock solution of dental sterilizing water according to the present invention, the effective chlorine concentration is of from 400 to 700 ppm instead of from 201 to 700 ppm.

In the stock solution of dental sterilizing water according to the present invention, the effective chlorine concentration is of from 500 to 700 ppm instead of from 201 to 700 ppm.

In the stock solution of dental sterilizing water according to the present invention, the effective chlorine concentration is of from 50 to 300 ppm instead of from 50 to 700 ppm and the stock solution is used for preventing a dental disease.

In the stock solution of dental sterilizing water according to the present invention, the pH is of 7 to 8 instead of 6.3 to 8.

A producing apparatus for producing dental sterilizing water is an apparatus for treating or preventing a dental disease by sterilizing periodontal pathogen, dental caries pathogen, or other oral bacteria, the producing apparatus comprising a stock solution tank for storing a stock solution containing sodium chloride and carbon dioxide and an electrolysis tank being connected to and communicating with the stock solution tank and for electrolyzing the stock solution, wherein the electrolysis tank is configured such that hypochlorous acid and sodium hydrogen carbonate are produced in predetermined concentrations by electrolyzing the stock solution so as to give an effective chlorine concentration of 50 to 700 ppm and a pH of 6.3 to 8.

In the producing apparatus for producing dental sterilizing water, the effective chlorine concentration is of 201 to 700 ppm, instead of 50 to 700 ppm and the producing apparatus is used for treating a dental disease.

In the producing apparatus for producing dental sterilizing water, the effective chlorine concentration is of 400 to 700 ppm, instead of 201 to 700 ppm.

In the producing apparatus for producing dental sterilizing water, the effective chlorine concentration is of 500 to 700 ppm, instead of 201 to 700 ppm.

In the producing apparatus for producing dental sterilizing water, the effective chlorine concentration is of 50 to 300 ppm, instead of 50 to 700 ppm and the producing apparatus is used for preventing a dental disease.

In the producing apparatus for producing dental sterilizing water, the pH is of 7 to 8, instead of 6.3 to 8.

As described in the background of art (WO 2007-72697), in the development of sterilizing water for periodontal disease, the present applicant has initially succeeded in the production of sterilizing water containing hypochlorous acid (HClO) at a high concentration by using carbon dioxide naturally dissolved in well water or tap water and has confirmed in a clinical test that the sterilizing water can annihilate periodontal pathogen.

However, as described above, even if hypochlorous acid can be produced at a high concentration, the sterilizing water cannot be brought into contact with bacteria if the biofilms are not destroyed. Consequently, another device for destroying the biofilms is necessary.

Actually, in the clinical practice of treating periodontal disease, biofilms must be physically destroyed with an ultrasonic scaler or laser beams. In the clinical practice of treating dental caries, biofilms formed on the surfaces of teeth must be physically removed by a tooth surface-cleaning method in which sodium hydrogen carbonate fine power and water are sprayed to the surfaces of teeth with compressed air.

Therefore, the present applicant has further studied on the destruction of biofilms and, as a result, has obtained new findings that sterilizing water can contain not only hypochlorous acid (HClO) but also sodium hydrogen carbonate (NaHCO₃) newly produced at high concentrations by producing the sterilizing water by preparing an aqueous solution as a stock solution containing sodium chloride (NaCl) and carbon dioxide and electrolyzing the stock solution such that the effective chlorine concentration is from 201 to 700 ppm and the pH is from 6.3 to 8. Furthermore, it was confirmed by the test results that though dental caries pathogen are thought to be difficult to be annihilated due to their very thick cell walls, the dental caries pathogen can be annihilated within about several to several tens seconds by only rinsing the inside of the oral cavity with the sterilizing water even under the circumstances that dental caries pathogen inhabit in the oral cavity, that is, under circumstances that dental caries pathogen are protected by biofilms, and also confirmed that periodontal pathogen can be annihilated within about several to several tens seconds by only injecting the sterilizing water into periodontal pockets.

This means that pathogenic bacteria can be completely sterilized by cooperation of sodium hydrogen carbonate and hypochlorous acid, that is, the destruction of biofilms with the sodium hydrogen carbonate at a high concentration and the annihilation of the bacteria in the destroyed biofilms with the hypochlorous acid at a high concentration. Therefore, this is a remarkable invention not only in the dental field but also in the entire medical field.

In addition, it has been confirmed that in the oral cavity portions other than periodontal pockets, such as tooth crown surfaces and tooth root surfaces at relatively shallow areas, where the amount of organic substances that cause a reduction in the hypochlorous acid concentration is relatively low, since sodium hydrogen carbonate destroys biofilms, oral bacteria inhabiting in the above-mentioned portions can be sufficiently sterilized, even if the effective chlorine concentration is lower than 201 ppm.

Furthermore, the dental sterilizing water according to the present invention can be used for preventing dental diseases as well as treating the dental diseases. In particular, by controlling the effective chlorine concentration to from 50 to 300 ppm, sufficient safety can be secured even in daily use by patients themselves, and therefore use in home is possible.

The dental diseases are diseases caused by oral bacteria and typically include a periodontal disease caused by periodontal pathogen and dental caries caused by dental caries pathogen, but are not limited such diseases and include diseases that are caused by bacteria inhabiting in the oral cavity.

In order to conduct electrolysis such that the effective chlorine concentration is from 201 to 700 ppm and the pH is from 6.3 to 8, the necessary content of sodium chloride is added, for example, 2 to 5% by mass. Since the amount of carbon dioxide naturally dissolved in water at a partial pressure of carbon dioxide in the air (380 ppm, annual average concentration of carbon dioxide in the air in Japan, an excerpt from "Rika Nenpyo, Kankyo-hen" (Chronological Scientific Tables, Environment), 2nd edition) is absolutely too low, it is necessary to increase the degree of solubility of carbon dioxide by forcibly dissolving carbon dioxide.

That is, in this description, the term "forcibly dissolving carbon dioxide" refers to that the degree of solubility of carbon dioxide is increased to a level higher than the concentration of carbon dioxide that can naturally dissolve (the degree of solubility at a partial pressure of carbon dioxide present in the air). Here, in a specific method of forcibly dissolving carbon dioxide, a stock solution may be prepared by any of the following methods (a) to (d), but in any of the methods, acids other than carbonic acid, such as hydrochloric acid and acetic acid, are not used at all. Therefore, the main parameter of conditions of stock solution composition is the additive amount of sodium chloride.

(a) Water is forced to pass through reverse osmosis membrane; sodium chloride is added to the passed water; and carbon dioxide gas is blown into the water or dry ice is added to the water before or after the step of adding sodium chloride or in the step of adding sodium chloride.

(b) Sodium chloride is added to pure water or distilled water; and carbon dioxide gas is blown into the water or dry ice is added to the water before or after the step of adding sodium chloride or in the step of adding sodium chloride.

(c) Water is forced to pass through reverse osmosis membrane; sodium chloride is added to the passed water; and the partial pressure of carbon dioxide being in contact with the passed water is increased to a level higher than that of carbon dioxide in the air before or after the step of adding sodium chloride or in the step of adding sodium chloride.

(d) Sodium chloride is added to pure water or distilled water; and the partial pressure of carbon dioxide being in contact with the pure water or the distilled water is increased to a level higher than that of carbon dioxide in the air before or after the step of adding sodium chloride or in the step of adding sodium chloride.

Here, in the above (a) and (c), the water as a component of the stock solution may be, for example, well water or tap water and is not required to be pure water. However, it is obvious that the use of pure water not containing calcium ions, magnesium ions and so on is better for preventing damage of electrodes of an electrolysis tank or a reduction in the electrode reaction.

In order to sterilize periodontal pathogen, dental caries pathogen, or other oral bacteria with the sterilizing water according to the present invention, for the dental caries pathogen, the sterilizing water is put into the oral cavity to be gargled in the oral cavity from several to several tens seconds, and for the periodontal pathogen, the sterilizing water is injected into the periodontal pockets.

By doing so, the sterilizing water, while destroying the biofilms, certainly sterilizes periodontal pathogen, dental caries pathogen, or other oral bacteria in a short period of time, though the sterilizing water gradually loses the bactericidal activity by oxidation of organic substances and other bacteria being present in the circumstance.

Here, the pH is adjusted to from 6.3 to 8. A pH of 6.3 or less may cause decalcification of teeth. Furthermore, in a pH lower than 6, the presence ratio of HCO₃⁻ in the concentration fractions of H₂CO₃, HCO₃⁻ and CO₃²⁻ is reduced, which makes it difficult to produce sodium hydrogen carbonate at a level that can destroy the biofilms. In a pH higher than 8, the presence ratio of HClO in the concentration fractions of Cl₂, HClO, and ClO⁻ is reduced, which makes it difficult to produce hypochlorous acid at a high concentration that can sterilize bacteria, in particular, dental caries pathogen.

In addition, the pH is desirably 7 or more. By doing so, lactic acid produced by dental caries pathogen is neutralized and the acidification in the oral cavity is prevented.

Furthermore, the effective chlorine concentration is adjusted to 50 ppm or more. When the concentration is lower than 50 ppm, it is difficult to sterilize oral bacteria even in the oral bacteria adhering to tooth crown surfaces or tooth root surfaces at shallow areas.

Furthermore, the effective chlorine concentration is adjusted to 201 ppm or more. When the concentration is 200 ppm or less, the oral bacteria inhabiting in the deep zones of periodontal pockets are not sterilized. In addition to the above, it is difficult to achieve the following conditions (i) to (iii).

(i) In general, the ratio of oral bacteria that form biofilms and inhabit therein is overwhelmingly larger than that of oral bacteria that inhabit in a floating state. In order to annihilate the bacteria in the biofilms, it is necessary to maintain sufficient bactericidal activity even after the oxidation of various organic substances and other bacteria being present in the circumstance. The bactericidal activity of hypochlorous acid at an amount of about several tens parts per million is too low to sterilize bacteria other than those adhering to tooth crown surfaces and tooth root surfaces at shallow areas.

(ii) Sterilization taking a long time, for example, over 60 seconds, has a risk to transfer hundreds of thousands of oral bacteria to the inside of the body (the inside of a blood vessel) to cause bacteremia and induce systemic illness. Therefore, bacteria must be annihilated within 30 seconds, if possible, within 10 seconds.

(iii) In biofilms, 300 to 400 types of oral bacteria parasitically propagate, while maintaining a certain proportion, to form bacterial flora. However, if the bacteria are substituted with other bacteria or the number of a bacterium that is usually low in the number is abnormally increased by some causes, a change in the bacterial flora, a phenomenon called microbial substitution, occurs. That is, if parts of dental caries pathogen and periodontal pathogen survive without being sterilized, the microbial substitution occurs so that the remaining bacteria drastically propagate. In order to avoid such a situation, all bacteria inhabiting in the biofilms must be annihilated.

In addition, the effective chlorine concentration is desirably 500 ppm or more. Within this concentration, even if the presence ratio of hypochlorous acid is decreased at a pH of around 8, the amount of hypochlorous acid can be ensured so as to be sufficient for the sterilization or the bacteriolysis of oral bacteria such as dental caries pathogen and periodontal pathogen.
On the other hand, the effective chlorine concentration is 700 ppm or less. A concentration higher than 700 ppm is not necessary for achieving sterilization and the above (i) to (iii).

Here, when the effective chlorine concentration is from 300 to 700 ppm, the sterilization or the bacteriolysis of various oral bacteria, in particular, periodontal pathogen and dental caries pathogen can be achieved within about 30 seconds. When the effective chlorine concentration is from 400 to 700 ppm, the sterilization or the bacteriolysis of the bacteria can be achieved within about 10 seconds.

### BRIEF DESCRIPTION OF DRAWINGS

[Fig. 1] Fig. 1 is a schematic diagram showing an apparatus for producing sterilizing water for dental treatment according to an embodiment.
[Fig. 2] Fig. 2 is a graph showing the presence ratio of hypochlorous acid.
[Fig. 3] Fig. 3 is a schematic diagram showing an apparatus for producing sterilizing water for dental treatment according to a modification.

### REFERENCE NUMERALS

51 producing apparatus for producing sterilizing water for dental treatment
52 stock solution
3 stock solution tank
5 electrolysis tank
6 discharge tube
57 dilution water
8 dilution water tank
11 deaeration module
14 tertiary produced water tank

### BEST MODES FOR CARRYING OUT THE INVENTION

Embodiments of dental sterilizing water according to the present invention, a method of producing the water according to the present invention, and an apparatus for producing the water according to the present invention will now be described with reference to the accompanying drawings. It is noted that substantially the same elements as those in existing technology are denoted by the same reference numerals, and descriptions thereof are omitted.

Sterilizing water for dental treatment as the dental sterilizing water according to an embodiment contains hypochlorous acid (HClO) and sodium hydrogen carbonate (NaHCO₃). The effective chlorine concentration is from 201 to 700 ppm, desirably from 400 to 700 ppm, and further desirably from 500 to 700 ppm, and the pH is 6.3 or higher and 8 or less, desirably 7 or higher and 8 or less. Dental diseases such as periodontal disease and dental caries can be cured by sterilizing periodontal pathogen, dental caries pathogen, or other oral bacteria with the sterilizing water.

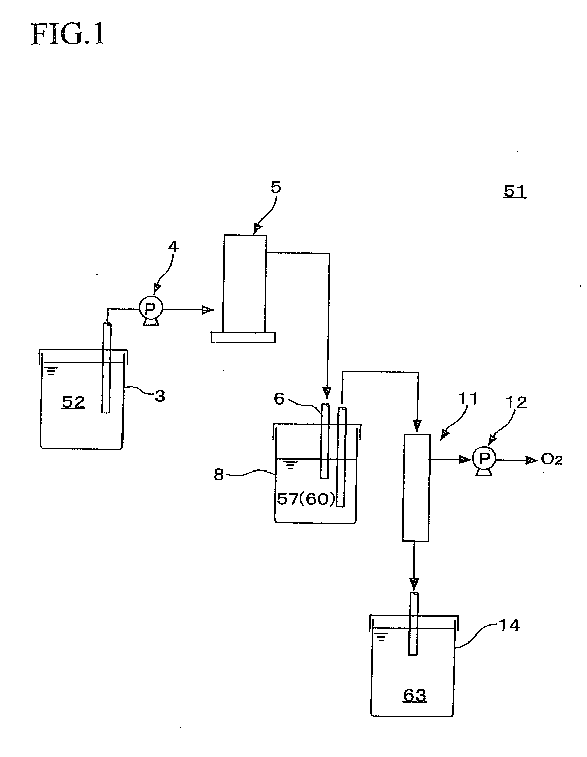

Figure 1 shows an apparatus for producing the sterilizing water for dental treatment according the embodiment.

As shown in the figure, the producing apparatus 51 for producing sterilizing water for dental treatment according to as described in the claims includes a stock solution tank 3 for storing a stock solution 52, a stroke pump 4 being connected to and communicating with the stock solution tank, an electrolysis tank 5 being connected to and communicating with the stroke pump, a discharge tube 6 being connected to and communicating with the electrolysis tank, and a dilution water tank 8 for storing dilution water 57. The dilution water tank 8 is relatively positioned with respect to the position of one end of the discharge tube 6 such that the end of the discharge tube 6 is below the water level of the dilution water 57 stored in the dilution water tank 8.

The stock solution 52 is prepared by any method described below and, in any method, does not contain any acid, such as hydrochloric acid or acetic acid, other than carbonic acid.

The dilution water 57 may be well water, tap water, pure water, or any other arbitrary water, but the pH of the water is properly determined so that the pH of produced sterilizing water is within the above-mentioned range.

The producing apparatus 51 as described herein further includes a deaeration module 11 having a water-injecting side communicating with secondary produced water 60 that is primary produced water diluted with dilution water 57 in the dilution water tank 8. The deaeration module removes dissolved oxygen in the secondary produced water 60 under a pressure reduced by a vacuum pump 12 and has a tertiary produced water tank 14 for storing tertiary produced water, as sterilizing water 63, that is the secondary produced water 60 from which dissolved oxygen is removed.

In addition, tubes provided to the producing apparatus 51 or an electromagnetic valve arbitrarily provided according to need may be deteriorated through oxidation by hypochlorous acid at a high concentration and, therefore, are desirably formed of fluorine.

In order to produce the sterilizing water 63 for dental treatment with the producing apparatus 51 for producing the sterilizing water as described in the claims, the composition conditions (mainly the additive amount of sodium chloride) of the stock solution 52, the operation conditions (for example, the voltage value and the current value) in electrolysis, and the dilution conditions (the dilution ratio and the pH of dilution water) are determined such that the effective chlorine concentration of the tertiary produced water is from 201 to 700 ppm, desirably from 400 to 700 ppm, and further desirably from 500 to 700 ppm, and the pH is from 6.3 to 8 and desirably from 7 to 8. The blended stock solution 52 is stored in the stock solution tank 3.

The additive amount of sodium chloride is, for example, 2 to 5% by mass.

The method for increasing the degree of solubility of carbon dioxide in a solvent, i.e., water passed through reverse osmosis membrane, pure water, or distilled water, may be as follows: a method for temporarily increasing the degree of solubility of carbon dioxide by forcibly mixing carbon dioxide into the solvent; a method by increasing the partial pressure of carbon dioxide being in contact with the solvent; or a method by decreasing the temperature of the solvent. Either the method by forcibly mixing carbon dioxide or the method by increasing the partial pressure of carbon dioxide is desirable in consideration of an increase in water temperature due to the heat produced in electrolysis.

The method for temporarily increasing the degree of solubility of carbon dioxide can be further classified into a method by blowing carbon dioxide gas and a method by adding dry ice. Here, the term "temporarily" refers to the case that since the partial pressure of carbon dioxide being in contact with the solvent is equal to that of carbon dioxide present in the air, in other words, since the mixing of carbon dioxide is performed under the atmospheric pressure, the degree of solubility of carbon dioxide is decreased according to the elapse of time due to the pressure equilibrium with the partial pressure of carbon dioxide present in the air, even if carbon dioxide is forcibly injected temporarily. In this case, it is necessary to promptly conduct electrolysis before that the degree of solubility of carbon dioxide is decreased.

The method for increasing the degree of solubility of carbon dioxide by increasing the partial pressure of carbon dioxide can be achieved by sealing a solvent, i.e., water passed through reverse osmosis membrane, pure water, or distilled water in an airtight tank and injecting carbon dioxide in the air space of the tank; or by blowing carbon dioxide gas into the solvent in the airtight tank; or adding dry ice to the solvent.

In this case, it is necessary to dissolve carbon dioxide at a certain partial pressure in the solvent and to conduct electrolysis by transferring the stock solution 52 to the electrolysis tank 5 while the partial pressure being maintained. Therefore, the stock solution tank 3, the stroke pump 4, and the electrolysis tank 5 may be constituted to be hermetic as a whole not to decrease the partial pressure of carbon dioxide.

From the above, carbon dioxide is forcibly dissolved by any method selected from the below.

(a-1) Tap water is forced to pass through reverse osmosis membrane; sodium chloride is added to the passed water; and carbon dioxide is forcibly dissolved in the passed water by blowing carbon dioxide gas into the water in the step of adding sodium chloride, before the addition step, or after the addition step.

(a-2) Tap water is forced to pass through reverse osmosis membrane; sodium chloride is added to the passed water; and carbon dioxide is forcibly dissolved in the passed water by adding dry ice to the water in the step of adding sodium chloride, before the addition step, or after the addition step.

(b-1) Sodium chloride is added to pure water or distilled water; and carbon dioxide is forcibly dissolved in the water by blowing carbon dioxide gas into the water in the step of adding sodium chloride, before the addition step, or after the addition step.

(b-2) Sodium chloride is added to pure water or distilled water; and carbon dioxide is forcibly dissolved in the water by adding dry ice to the water in the step of adding sodium chloride, before the addition step, or after the addition step.

(c) Water is forced to pass through reverse osmosis membrane; sodium chloride is added to the passed water; and carbon dioxide is dissolved in the passed water at a higher degree of solubility than that at the partial pressure of carbon dioxide in the air by increasing the partial pressure of carbon dioxide being in contact with the passed water to a level higher than that of carbon dioxide in the air.

(d) Sodium chloride is added to pure water or distilled water; and carbon dioxide is dissolved in the pure water or distilled water at a higher degree of solubility than that at the partial pressure of carbon dioxide in the air by increasing the partial pressure of carbon dioxide being in contact with the pure water or distilled water to a level higher than that of carbon dioxide in the air before or after the step of adding sodium chloride or in the step of adding sodium chloride.

The water to pass through reverse osmosis membrane may have any properties, but water purged to some extent is desirable from the standpoint of reducing the burden on the reverse osmosis membrane or a water purging system using the membrane or reducing the amount of water to be discarded as much as possible. For example, groundwater, tap water, or commercially available mineral water (commercially available water) can be used. Hereinafter, in the embodiment, tap water is used as the water that is forced to pass through reverse osmosis membrane.

In the case in which stock solution 52 is prepared by forcing tap water to pass through reverse osmosis membrane, an arbitrarily selected commercially available water purging system provided with reverse osmosis membrane may be used. Furthermore, in the case in which carbon dioxide is dissolved in the passed water, pure water, or distilled water under circumstances with a high partial pressure of carbon dioxide, a conventionally known apparatus for dissolving carbon dioxide can be arbitrarily used.

After the preparation of the stock solution 52, the stock solution 52 is weighed in an amount corresponding to one batch of sterilizing water, the weighed stock solution is stored in the stock solution tank 3, and dilution water 57 in an amount corresponding to one batch of the sterilizing water is stored in the dilution water tank 8. The amount of the dilution water 57 may be properly determined depending on the dilution ratio or the pH of the dilution water so as to correspond to one batch of the sterilizing water.

Then, the stock solution 52 is transferred to the electrolysis tank 5 with the stroke pump 4, and the electrolysis tank 5 is operated under predetermined operation conditions to electrolyze the stock solution 52.

Then, the primary produced water produced in the electrolysis tank 5 is injected into the dilution water 57 previously stored in the dilution water tank 8, through the discharge tube 6 being connected to and communicating with the electrolysis tank.

Here, the dilution water tank 8 is relatively positioned such that one end of the discharge tube 6 is below the water level of the dilution water 57 stored in the dilution water tank 8.

Therefore, the primary produced water is injected into the dilution water 57, without being brought into contact with air (external air), through the discharge tube 6. In addition, since the primary produced water is injected into the dilution water 57 weighed in advance, that is, injected by a batch system, the primary produced water is uniformly mixed with the dilution water 57, unlike the conventional mixing in piping.

Then, tertiary produced water is produced by forcing the secondary produced water 60 to pass through the deaeration module 11 for removing dissolved gas, in particular, dissolved oxygen. The tertiary produced water is stored as sterilizing water 33 in a tertiary produced water tank 14.

In order to sterilize periodontal pathogen, dental caries pathogen, or other oral bacteria with the sterilizing water 63 for dental treatment according to the prepared as defined in the claims, for the dental caries pathogen, for example, the sterilizing water is put into the oral cavity to be gargled in the oral cavity from several to several tens seconds, and for the periodontal pathogen, the sterilizing water may be injected into the periodontal pockets.

By bringing the sterilizing water 63 for dental treatment as defined in the claims into contact with oral bacteria by, for example, gargle in the oral cavity or injection into the periodontal pockets as described above, sodium hydrogen carbonate contained in the sterilizing water 63 destroys biofilms and, at the same time, hypochlorous acid certainly sterilizes target bacteria in a short period of time while gradually losing the bactericidal activity by oxidation of various organic substances and other bacteria present in the circumstance.

As described above, in the sterilizing water 63 for dental treatment according to the present invention, the method for producing the water according to the present invention, and the producing apparatus 51 embodiment, an aqueous solution containing sodium chloride and carbon dioxide is used as a stock solution, and the stock solution is electrolyzed such that the effective chlorine concentration is from 201 to 700 ppm, desirably from 400 to 700 ppm, and further desirably from 500 to 700 ppm, and the pH is from 6.3 to 8 and desirably from 7 to 8. This makes it possible to produce both sodium hydrogen carbonate at a high concentration sufficient for destroying biofilms and hypochlorous acid at a high concentration sufficient for sterilizing oral bacteria such as dental caries pathogen. Therefore, a remarkable effect that the sterilization of the oral bacteria or the bacteriolysis can be achieved within from several to several tens seconds is founded out, even without conducting destruction of biofilms in advance with a removing device, such as laser beams or an ultrasonic scaler, conventionally used.

In addition, in the method for producing the sterilizing water and the producing apparatus 51 according to the present invention, since carbon dioxide is forcibly dissolved, the effect that a high concentration of sodium hydrogen carbonate can be produced and thereby biofilms are destroyed is achieved. Since no addition of acid such as hydrochloric acid or acetic acid is required, sterilizing water without taste and odor can be produced. For example, even if the effective chlorine concentration is from 500 to 700 ppm, the effect that the perfectly sterilization of the oral bacteria or the bacteriolysis can be achieved within a short period of time of about from several to several tens seconds is founded out without causing any discomfort to patients.

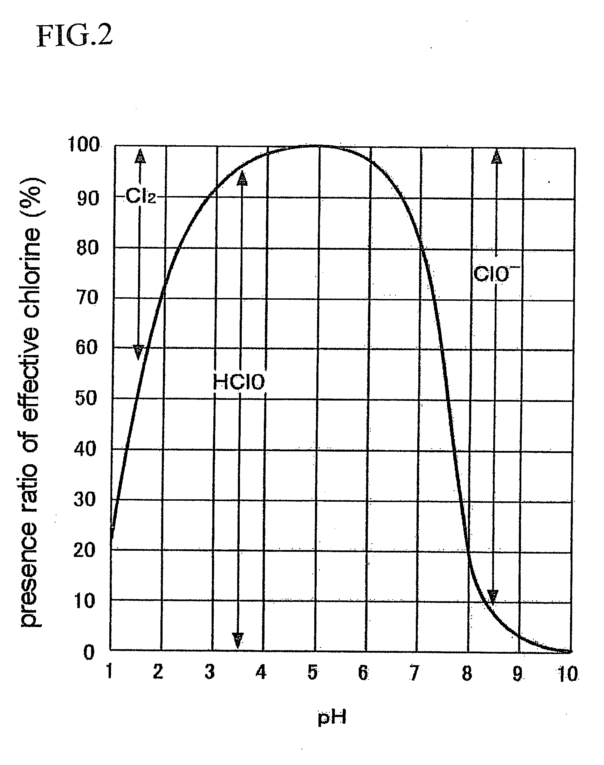

Figure 2 is a graph showing the presence ratio of effective chlorine, which is conventionally known (an excerpt from "Josui no Gijutsu" (Technique of Water Purging), published by Gihodo Shuppan Co., Ltd.). As obvious from the figure, it has been conventionally believed that the presence ratio of hypochlorous acid is drastically decreased at a pH of 7 or more and is 20% at a pH of 8.

However, a clinical test conducted by the present applicant (the details will be described below) gave the result that dental caries pathogen could be annihilated within a pH range of 6 to 8. Regarding dental caries pathogen, as described above, it is believed that in the dental field, denaturation of protein in bacterial cells by permeation through the cell walls, so-called bacteriolysis, is difficult even if hypochlorous acid is used, and even more, it is recognized that hypochlorous ion (ClO⁻) cannot destroy the cell walls of dental caries pathogen at all.

According to the sterilizing water 63 for use to the dental treatment according to the present invention, achieved are industrially noticeable effects that hypochlorous acid can annihilate bacteria in a pH range of 7 to 8 with sufficient bactericidal activity, which has not been conventionally noticed at all, and that bacteria in biofilms can be annihilated, without removing the biofilms in advance, by the synergistic effect with the biofilm-destroying function of sodium hydrogen carbonate being present in a high presence ratio in the above-mentioned pH range. Furthermore, in the annihilation of bacteria by the sterilizing water, the cell walls are broken and the protein in the bacterial cells is denatured, that is, the status of a so-called bacteriolysis is caused. Therefore, there is no risk of appearance of resistance bacteria.

Furthermore, in the producing apparatus 51 for producing sterilizing water for use in the dental treatment according to the as described in the claims, since the dilution water tank 8 is relatively positioned such that one end of the discharge tube 6 is below the water level of the dilution water 57 stored in the dilution water tank 8, the primary produced water is injected into the dilution water 57, without being brought into contact with air (external air). Consequently, even if the blend ratio of the stock solution 52 or the operation condition of the electrolysis tank 5 is different from those that are designed and thereby chlorine gas is produced, the chlorine gas is changed into a form of hypochlorous acid in the dilution water 57 having approximately neutral pH environment and does not have a risk of volatilizing into the air as chlorine gas.

In addition, since the primary produced water produced in the electrolysis tank 5 is injected into the dilution water 57 weighed in advance by a batch system, uniform mixing is possible, unlike the conventional mixing in piping, and thereby, the pH and the effective chlorine concentration of the secondary produced water 60 can be adjusted to designed levels.

Furthermore, in the method for producing the sterilizing water as defined in the claims and the producing apparatus 51 as described herein, the tertiary produced water 63 is produced as the sterilizing water by removing dissolved gas from the secondary produced water 60. Therefore, a phenomenon of forming foam in the oral cavity can be avoided from happening, and a situation of transferring oral bacteria to the inside of the body (the inside of a blood vessel) can be avoided from happening.

In the embodiment, the dissolved gas in the secondary produced water 60 is removed using the deaeration module 11, but the step of removing dissolved gas may be omitted provided that the dissolved gas concentration in the secondary produced water 60 is low and therefore there is not a risk of the foaming phenomenon. In such a case, the secondary produced water 60 is the sterilizing water.

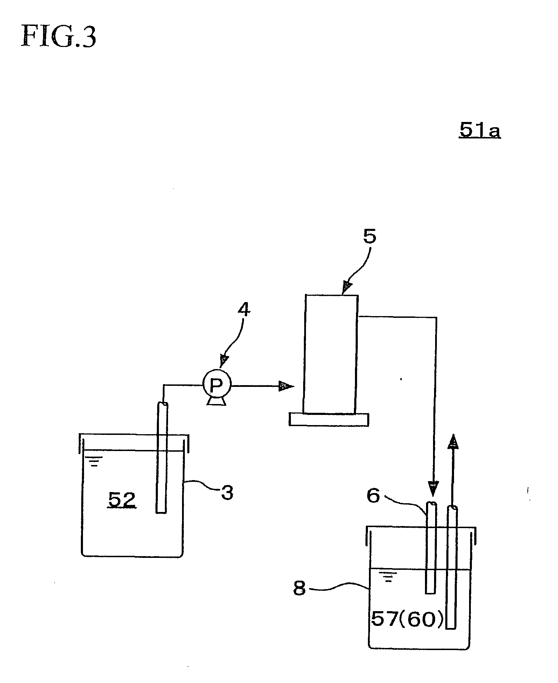

Figure 3 is a diagram showing a producing apparatus 51a that is used when the step of removing dissolved gas is omitted, wherein the deaeration module 11, the vacuum pump 12, and the tertiary produced water tank 14 are omitted from the producing apparatus 21.

In addition, in the embodiment, the stock solution 52 and the dilution water 57 are weighed in amounts that correspond to one batch of the sterilizing water, and the weighed stock solution and the weighed dilution water are stored in the stock solution tank 3 and the dilution water tank 8, respectively, in advance. Alternatively, if the stock solution 52 is stored in the stock solution tank 3 in advance in an amount larger than that corresponding to one batch of the sterilizing water, for example, an amount corresponding to several batches, a water level measuring device may be provided for weighing the stock solution 52 in an amount corresponding to one batch of the sterilizing water in each time. Such a water level measuring device can be properly configured with, for example, an ultrasound sensor or an electrode-type sensor.

Furthermore, in the embodiment, the stock solution is electrolyzed, followed by dilution to give sterilizing water (post-dilution). Alternatively, the stock solution may be diluted, and then the diluted water may be electrolyzed to give sterilizing water (pre-dilution). In such a modification, the dilution water tank 8 is omitted, and alternatively, a diluted stock solution tank for storing the diluted stock solution may be independently provided between the stock solution tank 3 and the electrolysis tank 5.

Furthermore, in one embodiment according to the present invention, the effective chlorine concentration is from 201 to 700 ppm. However, since the amount of organic substances that cause a reduction in concentration of hypochlorous acid is relatively low in the oral cavity at portions other than periodontal pockets, such as tooth crown surfaces and tooth root surfaces at relatively shallow areas, the oral bacteria that inhabit in such portions can be sufficiently sterilized even if the effective chlorine concentration is lower than 201 ppm. That is, even if the effective chlorine concentration is 50 ppm or more but less than 201 ppm, the sterilizing water can be used for treating dental diseases.

Furthermore, in one embodiment according to the present invention, the sterilizing water for dental treatment has been described as that for treating dental diseases. The dental sterilizing water according to the present invention is not limited to the therapeutic application, but can be applied to prevention of dental diseases.

In particular, by controlling the effective chlorine concentration to from 50 to 300 ppm, sufficient safety can be secured even in daily use by patients themselves, and therefore use in home is possible.

### EXAMPLE 1

### (Production of sterilizing water)

First, tap water was injected into a water purging system provided with reverse osmosis membrane, and then 3% by mass of sodium chloride and dry ice were added to the water passed through the reverse osmosis membrane to prepare a stock solution. The stock solution was diluted five times (pre-dilution).

Then, the diluted stock solution was electrolyzed in an electrolysis tank and sterilizing water was produced.

By the electrolysis in the above-described process, sterilizing water having a pH in the range of 6.3 to 8 and an effective chlorine concentration of 600 to 700 ppm was produced. In the measurement of the concentration of effective chlorine in the sterilizing water, since a meter and a test paper or reagent that can measure a concentration higher than 200 ppm were not available, the effective chlorine concentration was measured after doubling dilution was repeated twice.

In addition, as a control (standard reagent) for confirming the effect of 500 ppm of the sterilizing water, 40 ppm of sterilizing water was also prepared by the same procedure.

### EXAMPLE 2

### (Summary of clinical test using sterilizing water: periodontal pathogen)

A clinical test against periodontal pathogen was performed. In the test, the sterilizing water was injected into a periodontal pocket for treatment; then, a probe was inserted to the bottom of the periodontal pocket, while keeping the probe from being brought into contact with saliva, for sampling plaque adhering to the surface of a teeth root surface; the plaque was suspended in saline on a slide glass; and the suspension was covered with a cover glass and observed with a high-resolution phase-contrast microscope at 3600 magnification. Then, it was investigated whether the bacteria were sterilized or not by the observation with the microscope. Table 1 shows the test results.

**[Table 1]**

| | Patient No. | Properties of sterilizing water | | Main periodontal pathogen | possibility of bacteriolysis |
|---|---|---|---|---|---|
| | | pH | Effective chlorine conc. (ppm) | | |
| 1 | No. 2 | 7.5 | 627 | Treponema denticola Porphyromonas gingivalis | Ⓞ |
| 2 | No. 3 | 7.5 | 618 | Corynebacterium matruchotii Streptococcus sanguinis Tannerella forsythensis | Ⓞ |
| 3 | No. 6 | 7.5 | 625 | Treponema denticola Campylobacter rectus Porphyromonas gingivalis | Ⓞ |
| 4 | No. 7 | 7.6 | 675 | Tannerela forsythensis or Fusobacterium nucleatum Treponema denticola | Ⓞ |
| 5 | No. 12 | 7.7 | 690 | Treponema denticola Campylobacter rectus | Ⓞ |
| 6 | No. 16 | 7.7 | 690 | Prevotella intermedia | Ⓞ |
| 7 | No. 17 | 7.5 | 618 | Treponema denticola Tannerella forsythensis | Ⓞ |
| 8 | No. 19 | 7.2 | 603 | Treponema denticola Campylobacter rectus Capnocytophaga | Ⓞ |
| 9 | No. 20 | 7.5 | 627 | Eikenella corrodens Campylobacter rectus | Ⓞ |
| 10 | No. 25 | 7.6 | 675 | Porphyromonas gingivalis Treponema denticola Tannerella forsythensis or Fusobacterium nucleatum | Ⓞ |

As shown in the table, bacteriolysis of the periodontal pathogen of all patients was caused by the sterilizing water according to the present invention.

### (Summary of clinical test using sterilizing water: dental caries pathogen)

Next, a clinical test against dental caries pathogen was performed. In the test, the oral cavity was rinsed with the sterilizing water for 10 seconds; then, saliva was sampled; and the sampled saliva was investigated for the numbers of Streptococcus mutans, Streptococcus sobrinus, and Lactobacilli (per 1 ml of saliva). The test was carried out using "CAT 21 Fast" (short-time dental caries activity test) available from Morita Corporation.

The numbers of bacteria were counted for two cases: a culturing time of 20 minutes (37°C) and a culturing time of 24 hours (37°C). When the effective chlorine concentration was 40 ppm, the number of bacteria in the culturing time of 20 minutes was from 10² to 10³ (from safety range to caution range), and the number in the culturing time of 24 hours was from 10⁵ to 10⁶ (danger range). These test results show that an effective chlorine concentration of about 40 ppm cannot sufficiently sterilize dental caries pathogen.

The test results using the sterilizing water according to the present invention are shown in the following Table 2.

**[Table 2]**

| Patient No. | Before treatment (before gargle) | Properties during sterilizing | | After treatment (gargling for 10 sec.) |
|---|---|---|---|---|
| | | pH | Effective chlorine conc. (ppm) | |
| 1 | high danger range (+++) | 7.6 | 675 | safety range |
| 2 | danger range (++) | 7.5 | 627 | safety range |
| 3 | danger range (++) | 7.2 | 603 | safety range |
| 4 | caution range (+) | 7.5 | 618 | safety range |
| 5 | high danger range (+++) | 7.7 | 690 | safety range |
| 6 | danger range (++) | 7.7 | 690 | safety range |
| 7 | high danger range (+++) | 7.5 | 627 | safety range |
| 8 | high danger range (+++) | 7.5 | 618 | safety range |
| 9 | caution range (+) | 7.5 | 625 | safety range |
| 10 | danger range (++) | 7.6 | 675 | safety range |

As shown in the table, the number of bacteria after treatment with the sterilizing water according to the present invention was in safety range in all patients, and it was thereby confirmed that dental caries pathogen can be lysed. This is believed that the synergistic effect of the biofilm-destroying function of sodium hydrogen carbonate and the bactericidal activity of hypochlorous acid enables the annihilation of dental caries pathogen.

### EXAMPLE 3

### (Production of sterilizing water: Experiment 2)

### 1) Stock solution

The following four test solutions were prepared as stock solutions.

### Test solution A

Dry ice (5% (w/v)) was added to distilled water under atmospheric pressure and at room temperature to dissolve carbon dioxide constituting the dry ice in the distilled water (saturated carbonic acid solution). After a certain period of time, sodium chloride (0.6% (w/v)) was dissolved in the solution.

### Test solution B

The saturated carbonic acid solution as the intermediate of the test solution A was diluted five times with distilled water. After a certain period of time, sodium chloride (0.6% (w/v)) was dissolved in the solution.

### Test solution C

The saturated carbonic acid solution as the intermediate of the test solution A was diluted ten times with distilled water. After a certain period of time, sodium chloride (0.6% (w/v)) was dissolved in the solution.

### Test solution D

Distilled water was exposed with the air under atmospheric pressure and at room temperature to dissolve carbon dioxide in the air in the distilled water. Then, sodium chloride (0.6% (w/v)) was dissolved in the solution.

### 2) Test method

Four liters of the stock solution was put into an electrolysis tank without barrier membrane, and electrolysis was carried out at a direct current of 2.8 A.

### 3) Results

Table 3 shows the test results.

**[Table 3]**

| | Test solution A | | Test solution B | | Test solution C | | Test solution D | |
|---|---|---|---|---|---|---|---|---|
| Electrolysis time (min) | pH | Effective chlorine | pH | Effective chlorine | pH | Effective chlorine | pH | Effective chlorine |
| 0 (before electrolysis) | 3.8 | 0 | 4.2 | 0 | 4.8 | 0 | 5.3 | 0 |
| 14 | 4.9 | 110 | 5.7 | 115 | 5.8 | 115 | 8.8 | 110 |
| 20 | 5.5 | 215 | 6.2 | 215 | 6.5 | 210 | 9.1 | 220 |
| 30 | 5.7 | 335 | 6.6 | 330 | 6.8 | 325 | 9.2 | 315 |
| 40 | 6.1 | 415 | 6.8 | 435 | 7.2 | 425 | 9.2 | 420 |
| 50 | 6.2 | 510 | 7 | 525 | 7.4 | 520 | 9.2 | 510 |
| 60 | 6.3 | 630 | 7.2 | 625 | 7.6 | 625 | 9.2 | 620 |
| 70 | 6.4 | 705 | 7.4 | 695 | 7.8 | 700 | 9.2 | 710 |
| 80 | 6.5 | 790 | 7.4 | 785 | 7.9 | 790 | 9.2 | 780 |
| 90 | 6.6 | 845 | 7.5 | 830 | 7.9 | 825 | 9.2 | 850 |

### (Effective chlorine: mg/kg)

As shown in the table, the pH levels of the test solutions A to C employing saturated carbonic acid solution were in the range of 6 to 8, in which sufficient concentrations of hypochlorous acid and sodium hydrogen carbonate can be kept. On the other hand, the pH of the test solution D, in which carbon dioxide in the air was naturally dissolved, was 9.2. Therefore, it is suggested that the method by naturally dissolving carbon dioxide present in the air is difficult to produce both hypochlorous acid and sodium hydrogen carbonate at sufficient concentrations.

## Claims

1. Dental sterilizing water for use in treating or preventing a dental disease by sterilizing periodontal pathogen, dental caries pathogen, or other oral bacteria, the dental sterilizing water having an effective chlorine concentration of 50 to 700 ppm and a pH of 6.3 to 8 and containing hypochlorous acid and sodium hydrogen carbonate, wherein the dental sterilizing water is produced by preparing an aqueous solution as a stock solution, wherein the stock solution is prepared by any of the following methods:
(i) by forcing water to pass through reverse osmosis membrane; adding sodium chloride to the passed water; and blowing carbon dioxide gas into the water or adding dry ice to the water before or after the step of adding sodium chloride or in the step of adding sodium chloride; or
(ii) by adding sodium chloride to pure water or distilled water; and blowing carbon dioxide gas into the water or adding dry ice to the water before or after the step of adding sodium chloride or in the step of adding sodium chloride; or
(iii) by forcing water to pass through reverse osmosis membrane; adding sodium chloride to the passed water; and increasing the partial pressure of carbon dioxide being in contact with the passed water to a level higher than that of carbon dioxide in the air before or after the step of adding sodium chloride or in the step of adding sodium chloride; or
(iv) by adding sodium chloride to pure water or distilled water; and increasing the partial pressure of carbon dioxide being in contact with the pure water or the distilled water to a level higher than that of carbon dioxide in the air before or after the step of adding sodium chloride or in the step of adding sodium chloride; and then electrolyzing the stock solution.

2. The dental sterilizing water for use according to Claim 1, having an effective chlorine concentration of 201 to 700 ppm, preferably of 400 to 700 ppm, more preferably of 500 to 700 ppm and being used for treating a dental disease.

3. The dental sterilizing water for use according to Claim 1, having an effective chlorine concentration of 50 to 300 ppm and being used for preventing a dental disease.

4. The dental sterilizing water for use according to any one of Claims 1 to 3, having a pH of 7 to 8.

5. A method for producing dental sterilizing water for treating or preventing a dental disease by sterilizing periodontal pathogen, dental caries pathogen, or other oral bacteria, the method comprising the steps of preparing an aqueous solution as a stock solution containing sodium chloride and carbon dioxide; and electrolyzing the stock solution such that the effective chlorine concentration is from 50 to 700 ppm, the pH is from 6.3 to 8, and hypochlorous acid and sodium hydrogen carbonate are produced, wherein the stock solution is prepared by any of the following methods:
(i) by forcing water to pass through reverse osmosis membrane; adding sodium chloride to the passed water; and blowing carbon dioxide gas into the water or adding dry ice to the water before or after the step of adding sodium chloride or in the step of adding sodium chloride; or
(ii) by adding sodium chloride to pure water or distilled water; and blowing carbon dioxide gas into the water or adding dry ice to the water before or after the step of adding sodium chloride or in the step of adding sodium chloride; or
(iii) by forcing water to pass through reverse osmosis membrane; adding sodium chloride to the passed water; and increasing the partial pressure of carbon dioxide being in contact with the passed water to a level higher than that of carbon dioxide in the air before or after the step of adding sodium chloride or in the step of adding sodium chloride; or
(iv) by adding sodium chloride to pure water or distilled water; and increasing the partial pressure of carbon dioxide being in contact with the pure water or the distilled water to a level higher than that of carbon dioxide in the air before or after the step of adding sodium chloride or in the step of adding sodium chloride.

6. The method for producing dental sterilizing water according to Claim 5, wherein the effective chlorine concentration is from 201 to 700 ppm, preferably from 400 to 700 ppm, more preferably from 500 to 700 ppm and being used for treating a dental disease.

7. The method for producing dental sterilizing water according to Claim 5, wherein the effective chlorine concentration is from 50 to 300 ppm and being used for preventing a dental disease.

8. The method for producing dental sterilizing water according to any one of Claims 5 to 7, wherein the pH is from 7 to 8.

9. A stock solution of dental sterilizing water for use in treating or preventing a dental disease by sterilizing periodontal pathogen, dental caries pathogen, or other oral bacteria, the stock solution containing sodium chloride and carbon dioxide; and giving an effective chlorine concentration of 50 to 700 ppm, a pH of 6.3 to 8, and producing hypochlorous acid and sodium hydrogen carbonate by electrolysis, wherein the stock solution is prepared by any of the following methods:
(i) by forcing water to pass through reverse osmosis membrane; adding sodium chloride to the passed water; and blowing carbon dioxide gas into the water or adding dry ice to the water before or after the step of adding sodium chloride or in the step of adding sodium chloride; or
(ii) by adding sodium chloride to pure water or distilled water; and blowing carbon dioxide gas into the water or adding dry ice to the water before or after the step of adding sodium chloride or in the step of adding sodium chloride; or
(iii) by forcing water to pass through reverse osmosis membrane; adding sodium chloride to the passed water; and increasing the partial pressure of carbon dioxide being in contact with the passed water to a level higher than that of carbon dioxide in the air before or after the step of adding sodium chloride or in the step of adding sodium chloride; or
(iv) by adding sodium chloride to pure water or distilled water; and increasing the partial pressure of carbon dioxide being in contact with the pure water or the distilled water to a level higher than that of carbon dioxide in the air before or after the step of adding sodium chloride or in the step of adding sodium chloride.

10. The stock solution of dental sterilizing water for use according to Claim 9, giving an effective chlorine concentration of 201 to 700 ppm, preferably of 400 to 700 ppm, more preferably of 500 to 700 ppm and being used for treating a dental disease.

11. The stock solution of dental sterilizing water for use according to Claim 9, wherein the effective chlorine concentration is of 50 to 300 ppm and being used for preventing a dental disease.

12. The stock solution of dental sterilizing water for use according to any one of Claims 9 to 11, wherein the pH is of 7 to 8.

## Patentansprüche

1. Zahnsterilisierungswasser zur Verwendung bei der Behandlung oder Vorbeugung einer Zahnkrankheit durch Sterilisieren eines parodontalen Pathogens, Zahnkaries-Pathogens oder anderer oraler Bakterien, wobei das Zahnsterilisierungswasser eine wirksame Chlorkonzentration von 50 bis 700 ppm und einen pH von 6,3 bis 8 besitzt und Hypochlorsäure und Natriumhydrogencarbonat enthält, wobei das Zahnsterilisierungswasser durch Zubereiten einer wässrigen Lösung als eine Stocklösung hergestellt wird, wobei die Stocklösung durch irgendeine der folgenden Verfahren hergestellt ist:
(i) Durch Zwingen von Wasser, eine Umkehrosmose-Membran zu durchströmen; Zugeben von Natriumchlorid zu dem durchgeströmten Wasser; und Blasen von Kohlendioxidgas in das Wasser oder Zugeben von Trockeneis zu dem Wasser vor oder nach dem Schritt der Zugabe von Natriumchlorid oder beim Schritt des Zugebens von Natriumchlorid; oder
(ii) Durch Zugeben von Natriumchlorid zu reinem Wasser oder destilliertem Wasser; und Blasen von Kohlendioxidgas in das Wasser oder Zugeben von Trockeneis zu dem Wasser vor oder nach dem Schritt des Zugebens von Natriumchlorid oder beim Schritt des Zugebens von Natriumchlorid; oder
(iii) durch Zwingen von Wasser, eine Umkehrosmose-Membran zu durchströmen; Zugeben von Natriumchlorid zu dem durchgeströmten Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem durchgeströmten Wasser ist, auf ein Niveau, das höher als das von Kohlendioxid in der Luft ist; oder
(iv) durch Zugeben von Natriumchlorid zu reinem Wasser oder destilliertem Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem reinen Wasser oder dem destillierten Wasser ist, auf ein Niveau, das höher als das von Kohlendioxid in der Luft ist;
und dann Elektrolysieren der Stocklösung.

2. Zahnsterilisierungswasser zur Verwendung gemäß Anspruch 1, mit einer wirksamen Chlorkonzentration von 201 bis 700 ppm, bevorzugt von 400 bis 700 ppm, mehr bevorzugt von 500 bis 700 ppm, und welches bei der Behandlung einer Zahnkrankheit verwendet wird.

3. Zahnsterilisierungswasser zur Verwendung gemäß Anspruch 1, mit einer wirksamen Chlorkonzentration von 50 bis 300 ppm, und welches bei der Vorbeugung einer Zahnkrankheit verwendet wird.

4. Zahnsterilisierungswasser zur Verwendung gemäß irgendeinem der Ansprüche 1 bis 3, mit einem pH von 7 bis 8.

5. Verfahren zur Herstellung von Zahnsterilisierungswasser bei der Behandlung oder Vorbeugung einer Zahnkrankheit durch Sterilisieren von parodontalem Pathogen, Zahnkaries-Pathogen oder anderen oralen Bakterien, wobei das Verfahren die Schritte des Zubereitens einer wässrigen Lösung als eine Stocklösung enthaltend Natriumchlorid und Kohlendioxid umfasst; und derartiges Elektrolysieren der Stocklösung, dass die wirksame Chlorkonzentration 50 bis 700 ppm beträgt, der pH 6,3 bis 8 ist, und Hydrochlorsäure und Natriumhydrogencarbonat hergestellt werden, wobei die Stocklösung durch irgendeines der folgenden Verfahren hergestellt wird:
(i) durch Zwingen von Wasser, eine Umkehrosmose-Membran zu durchströmen; Zugeben von Natriumchlorid zu dem durchgeströmten Wasser; und Blasen von Kohlendioxidgas in das Wasser oder Zugeben von Trockeneis zu dem Wasser vor oder nach dem Schritt des Zugebens von Natriumchlorid oder beim Schritt des Zugebens von Natriumchlorid; oder
(ii) durch Zugeben von Natriumchlorid zu reinem Wasser oder destilliertem Wasser; und Blasen von Kohlendioxidgas in das Wasser oder Zugeben von Trockeneis zu dem Wasser vor oder nach dem Schritt des Zugebens von Natriumchlorid oder beim Schritt des Zugebens von Natriumchlorid; oder
(iii) durch Zwingen von Wasser, eine Umkehrosmose-Membran zu durchströmen; Zugeben von Natriumchlorid zu dem durchgeströmten Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem durchgeströmten Wasser ist, auf ein Niveau, das höher als das von Kohlendioxid in der Luft ist; oder
(iv) durch Zugeben von Natriumchlorid zu reinem Wasser oder destilliertem Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem reinen Wasser oder dem destillierten Wasser ist, auf ein Niveau, das höher ist als das von Kohlendioxid in der Luft.

6. Verfahren zur Herstellung von Zahnsterilisierungswasser gemäß Anspruch 5, wobei die wirksame Chlorkonzentration 201 bis 700 ppm beträgt, bevorzugt 400 bis 700 ppm, mehr bevorzugt 500 bis 700 ppm, und wobei sie bei der Behandlung einer Zahnkrankheit verwendet wird.

7. Verfahren zur Herstellung von Zahnsterilisierungswasser gemäß Anspruch 5, wobei die wirksame Chlorkonzentration 50 bis 300 ppm beträgt und bei der Vorbeugung einer Zahnkrankheit verwendet wird.

8. Verfahren zur Herstellung von Zahnsterilisierungswasser gemäß irgendeinem der Ansprüche 5 bis 7, wobei der pH 7 bis 8 ist.

9. Zahnsterilisierungswasser-Stocklösung zur Verwendung bei Behandlung oder Vorbeugung einer Zahnkrankheit durch Sterilisieren von parodontalem Pathogen, Zahnkaries-Pathogen, oder anderen oralen Bakterien, wobei die Stocklösung Natriumchlorid und Kohlendioxid enthält; und eine wirksame Chlorkonzentration von 50 bis 700 ppm ergibt, einen pH von 6,3 bis 8, und Hypochlorsäure und Natriumhydrogencarbonat durch Elektrolyse herstellt, wobei die Stocklösung durch irgendeines der folgenden Verfahren hergestellt wird:
(i) durch Zwingen von Wasser, eine Umkehrosmose-Membran zu durchströmen; Zugeben von Natriumchlorid zu dem durchgeströmten Wasser; und Blasen von Kohlendioxidgas in das Wasser oder Zugeben von Trockeneis zu dem Wasser vor oder nach dem Schritt des Zugebens von Natriumchlorid oder beim Schritt des Zugebens von Natriumchlorid; oder
(ii) durch Zugeben von Natriumchlorid zu reinem Wasser oder destilliertem Wasser; und Blasen von Kohlendioxidgas in das Wasser oder Zugeben von Trockeneis zu dem Wasser vor oder nach dem Schritt des Zugebens von Natriumchlorid oder beim Schritt des Zugebens von Natriumchlorid; oder
(iii) durch Zwingen von Wasser, eine Umkehrosmose-Membran zu durchströmen; Zugeben von Natriumchlorid zu dem passierten Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem durchgeströmten Wasser ist, auf ein Niveau, das höher als das von Kohlendioxid in der Luft ist; oder
(iv) durch Zugeben von Natriumchlorid zu reinem Wasser oder destilliertem Wasser; und vor oder nach dem Schritt des Zugebens von Natriumchlorid oder beim Schritt des Zugebens von Natriumchlorid Erhöhen des Partialdrucks von Kohlendioxid, welches in Kontakt mit dem reinen Wasser oder dem destillierten Wasser ist, auf ein Niveau, das höher als das von Kohlendioxid in der Luft ist.

10. Zahnsterilisierungswasser-Stocklösung zur Verwendung gemäß Anspruch 9, ergebend eine wirksame Chlorkonzentration von 201 bis 700 ppm, bevorzugt von 400 bis 700 ppm, mehr bevorzugt von 500 bis 700 ppm, und welche bei der Behandlung einer Zahnkrankheit verwendet wird.

11. Zahnsterilisierungswasser-Stocklösung zur Verwendung gemäß Anspruch 9, wobei die wirksame Chlorkonzentration 50 bis 300 ppm beträgt und zur Vorbeugung einer Zahnkrankheit verwendet wird.

12. Zahnsterilisierungswasser-Stocklösung zur Verwendung gemäß irgendeinem der Ansprüche 9 bis 11, wobei der pH 7 bis 8 ist.

## Revendications

1. Eau de stérilisation dentaire pour utilisation dans le traitement ou la prévention d'une affection dentaire par stérilisation des agents pathogènes périodontiques, des agents pathogènes de caries dentaires ou d'autres bactéries buccales, l'eau de stérilisation dentaire ayant une concentration de chlore efficace de 50 à 700 ppm et un pH de 6,3 à 8 et contenant de l'acide hypochloreux et de l'hydrogénocarbonate de sodium, dans laquelle l'eau de stérilisation dentaire est produite en préparant une solution aqueuse comme solution mère, dans laquelle la solution mère est préparée par l'un quelconque des procédés suivants consistant à :
(i) obliger de l'eau à passer à travers une membrane d'osmose inverse ; ajouter du chlorure de sodium à l'eau qui s'en écoule ; et souffler de l'anhydride carbonique gazeuse dans l'eau ou ajouter de la glace sèche à l'eau avant ou après l'étape d'addition de chlorure de sodium ou au cours de l'étape d'addition de chlorure de sodium ; ou
(ii) ajouter du chlorure de sodium à de l'eau pure ou à de l'eau distillée ; et souffler de l'anhydride carbonique gazeuse dans l'eau ou ajouter de la glace sèche à l'eau avant ou après l'étape d'addition du chlorure de sodium ou au cours de l'étape d'addition du chlorure de sodium ; ou
(iii) obliger de l'eau à passer à travers une membrane d'osmose inverse ; ajouter du chlorure de sodium à l'eau qui s'en écoule ; et augmenter la pression partielle de l'anhydride carbonique qui est en contact avec l'eau qui s'en écoule à un niveau plus élevé que celui de l'anhydride carbonique dans l'air avant ou après l'étape d'addition de chlorure de sodium ou au cours de l'étape d'addition du chlorure de sodium ; ou
(iv) ajouter du chlorure de sodium à de l'eau pure ou à de l'eau distillée ; et augmenter la pression partielle de l'anhydride carbonique en contact avec l'eau pure ou l'eau distillée à un niveau supérieur à celui de l'anhydride carbonique dans l'air avant ou après l'étape d'addition de chlorure de sodium ou au cours de l'étape d'addition de chlorure de sodium ; et ensuite électrolyser la solution mère.

2. Eau de stérilisation dentaire pour utilisation selon la revendication 1, ayant une concentration de chlore efficace de 201 à 700 ppm, de préférence de 400 à 700 ppm, mieux encore de 500 à 700 ppm, et utilisée pour traiter une affection dentaire.

3. Eau de stérilisation dentaire pour utilisation selon la revendication 1, ayant une concentration de chlore efficace de 50 à 300 ppm et utilisée pour prévenir une affection dentaire.

4. Eau de stérilisation dentaire pour utilisation selon l'une quelconque des revendications 1 à 3, ayant un pH de 7 à 8.

5. Procédé de production d'eau de stérilisation dentaire pour le traitement ou la prévention d'une affection dentaire par stérilisation des agents pathogènes périodontiques, des agents pathogènes de caries dentaires ou d'autres bactéries buccales, le procédé comprenant les étapes consistant à préparer une solution aqueuse comme solution mère contenant du chlorure de sodium et de l'anhydride carbonique et à électrolyser la solution mère de sorte que la concentration de chlore efficace soit de 50 à 700 ppm, que le pH soit de 6,3 à 8 et que de l'acide hypochloreux et de l'hydrogénocarbonate de sodium soient produits, dans lequel la solution mère est préparée par l'un quelconque des procédés suivants consistant à :
(i) obliger de l'eau à passer à travers une membrane d'osmose inverse ; ajouter du chlorure de sodium à l'eau qui s'en écoule ; et souffler de l'anhydride carbonique gazeuse dans l'eau ou ajouter de la glace sèche à l'eau avant ou après l'étape d'addition de chlorure de sodium ou au cours de l'étape d'addition de chlorure de sodium ; ou
(ii) ajouter du chlorure de sodium à de l'eau pure ou à de l'eau distillée ; et souffler de l'anhydride carbonique gazeuse dans l'eau ou ajouter de la glace sèche à l'eau avant ou après l'étape d'addition du chlorure de sodium ou au cours de l'étape d'addition du chlorure de sodium ; ou
(iii) obliger de l'eau à passer à travers une membrane d'osmose inverse ; ajouter du chlorure de sodium à l'eau qui s'en écoule ; et augmenter la pression partielle de l'anhydride carbonique qui est en contact avec l'eau qui s'en écoule à un niveau plus élevé que celui de l'anhydride carbonique dans l'air avant ou après l'étape d'addition de chlorure de sodium ou au cours de l'étape d'addition du chlorure de sodium ; ou
(iv) ajouter du chlorure de sodium à de l'eau pure ou à de l'eau distillée ; et augmenter la pression partielle de l'anhydride carbonique en contact avec l'eau pure ou l'eau distillée à un niveau supérieur à celui de l'anhydride carbonique dans l'air avant ou après l'étape d'addition de chlorure de sodium ou au cours de l'étape d'addition de chlorure de sodium.

6. Procédé de production d'eau de stérilisation dentaire selon la revendication 5, dans lequel la concentration de chlore efficace est de 201 à 700 ppm, de préférence de 400 à 700 ppm, mieux encore de 500 à 700 ppm, et utilisée pour traiter une affection dentaire.

7. Procédé de production d'eau de stérilisation dentaire selon la revendication 5, dans lequel la concentration de chlore efficace est de 50 à 300 ppm et utilisée pour prévenir une affection dentaire.

8. Procédé de production d'eau de stérilisation dentaire selon l'une quelconque des revendications 5 à 7, dans lequel le pH est de 7 à 8.

9. Solution mère d'eau de stérilisation dentaire pour utilisation dans le traitement ou la prévention d'une affection dentaire par stérilisation des agents pathogènes périodontiques, des agents pathogènes de caries dentaires ou d'autres bactéries buccales, la solution mère contenant du chlorure de sodium et de l'anhydride carbonique ; et donnant une concentration de chlore efficace de 50 à 700 ppm, un pH de 6,3 à 8 et produisant de l'acide hypochloreux et de l'hydrogénocarbonate de sodium par électrolyse, dans laquelle la solution mère est préparée par l'un quelconque des procédés suivants consistant à :
(i) obliger de l'eau à passer à travers une membrane d'osmose inverse ; ajouter du chlorure de sodium à l'eau qui s'en écoule ; et souffler de l'anhydride carbonique gazeuse dans l'eau ou ajouter de la glace sèche à l'eau avant ou après l'étape d'addition de chlorure de sodium ou au cours de l'étape d'addition de chlorure de sodium ; ou
(ii) ajouter du chlorure de sodium à de l'eau pure ou à de l'eau distillée ; et souffler de l'anhydride carbonique gazeuse dans l'eau ou ajouter de la glace sèche à l'eau avant ou après l'étape d'addition du chlorure de sodium ou au cours de l'étape d'addition du chlorure de sodium ; ou
(iii) obliger de l'eau à passer à travers une membrane d'osmose inverse ; ajouter du chlorure de sodium à l'eau qui s'en écoule ; et augmenter la pression partielle de l'anhydride carbonique qui est en contact avec l'eau qui s'en écoule à un niveau plus élevé que celui de l'anhydride carbonique dans l'air avant ou après l'étape d'addition de chlorure de sodium ou au cours de l'étape d'addition du chlorure de sodium ; ou
(iv) ajouter du chlorure de sodium à de l'eau pure ou à de l'eau distillée ; et augmenter la pression partielle de l'anhydride carbonique en contact avec l'eau pure ou l'eau distillée à un niveau supérieur à celui de l'anhydride carbonique dans l'air avant ou après l'étape d'addition de chlorure de sodium ou au cours de l'étape d'addition de chlorure de sodium.

10. Solution mère d'eau de stérilisation dentaire pour utilisation selon la revendication 9, donnant une concentration de chlore efficace de 201 à 700 ppm, de préférence de 400 à 700 ppm, mieux encore de 500 à 700 ppm et utilisée pour traiter une affection dentaire.

11. Solution mère d'eau de stérilisation dentaire pour utilisation selon la revendication 9, dans laquelle la concentration de chlore efficace est de 50 à 300 ppm et utilisée pour prévenir une affection dentaire.

12. Solution mère d'eau de stérilisation dentaire pour utilisation selon l'une quelconque des revendications 9 à 11, dans laquelle le pH est de 7 à 8.
